# EUROPEAN PATENT APPLICATION

(11) **EP 2 837 688 A1**
(43) Date of publication of application: **18.02.2015**
(21) Application number: 13776232.4
(22) Date of filing: 12.04.2013
(51) Int. Cl.: C12P 13/10, C12N 15/09

(54) **METHOD FOR PRODUCING AMINO ACID**

(30) Priority: 13.04.2012 JP 2012091918
(71) Applicant: Kyowa Hakko Bio Co., Ltd., Tokyo 100-8185 (JP)
(72) Inventor: MITSUHASHI, Satoshi, Ibaraki 305-0841 (JP)
(74) Representative: Grünecker, Kinkeldey, Stockmair & Schwanhäusser
(86) International application number: PCT/JP2013/061060
(87) International publication number: WO 2013/154182

(57) **Abstract**

At least one amino acid selected from the group consisting of L-arginine, L-citrulline and L-ornithine can be produced with high efficiency by culturing a coryneform bacterium in a culture medium to produce and accumulate the amino acid in a culture and then collecting the amino acid from the culture, wherein the coryneform bacterium is produced by introducing a deletion, substitution or addition of at least one nucleotide into at least one gene which is present in chromosomal DNA of a parent strain and encodes a protein having an L-arginine, L-citrulline or L-ornithine uptake activity so that the activity of uptake of the amino acid in the coryneform bacterium is reduced or lost compared with that in the parent strain, and can produce the amino acid.

## Description

### Technical Field

The present invention relates to a process for producing at least one amino acid selected from the group consisting of L-arginine, L-citrulline, and L-ornithine using a coryneform bacterium which has an ability to produce and accumulate the amino acid.

### Background Art

It has been known that in a fermentative production of an amino acid, when the activity of uptake of the amino acid into a cell is reduced or lost, the amino acid excreted outside of the cell of a microorganism can be prevented from being taken up into the cell again and metabolized, and therefore, the productivity thereof is improved.

For example, with respect to L-glutamic acid, a process for producing L-glutamic acid using Corynebacterium glutamicum, in which the activity of uptake of L-glutamic acid into the cell is reduced or lost has been reported (Patent Document 1). Further, with respect to an aromatic amino acid, a process for producing an aromatic amino acid using Corynebacterium glutamicum, in which the activity of uptake of the aromatic amino acid into the cell is reduced or lost has been reported (Patent Document 2).

In Escherichia coli, three types of periplasmic binding protein-dependent proteins which take up L-arginine into the cell were identified as proteins having the activity of uptake of the amino acid into a cell (Non-Patent Document 1).

In coryneform bacteria, proteins having the activity of uptake of an amino acid into the cell with respect to L-methionine and L-alanine (Non-Patent Document 2), L-glutamic acid (Non-Patent Documents 3 and 4), L-isoleucine (Non-Patent Document 5), an aromatic amino acid (Non-Patent Document 6), L-lysine (Non-Patent Document 7) or the like were reported.

However, it is only described in Non-Patent Document 8 that NCg11278, NCg11277, and NCg11276 in Corynebacterium glutamicum are designated as BAB98725, BAB98724, BAB98723, respectively, and are classified in the ATP-binding Cassette (ABC) superfamily based on the homology to known amino acid sequences, and it has not been reported that these proteins have the activity of amino acid uptake. In addition, it has not been reported that the productivity of amino acids is improved by causing reduction or loss of the activity of these proteins.

### Related Art Documents

### Patent Documents

Patent Document 1: JP-A-2000-270872
Patent Document 2: Japanese Patent No. 3036819

### Non-Patent Documents

Non-Patent Document 1: THE JOURNAL OF MOLECULAR BIOLOGY, 2007, vol. 373, pp. 251-267
Non-Patent Document 2: BIOCHEMISTRY, 2008, vol. 47, No. 48, pp. 12698-12709
Non-Patent Document 3: APPLLIED MICROBIOLOGY AND BIOTECHNOLOGY, 2003, vol. 60, No. 6, pp. 738-742
Non-Patent Document 4: THE JOURNAL OF BACTERIOLOGY, 1995, vol. 177, No. 5, pp. 1152-1158
Non-Patent Document 5: ARCHIVES OF MICROBIOLOGY, 1998, vol. 169, No. 4, pp. 303-312
Non-Patent Document 6: THE JOURNAL OF BACTERIOLOGY, 1995, vol. 177, No. 20, pp. 5991-5993
Non-Patent Document 7: MOLECULAR MICROBIOLOGY, 1991, vol. 5, No. 12, pp. 2995-3005
Non-Patent Document 8: HANDBOOK OF CORYNEBACTERIUM GLUTAMICUM, 2005, p. 165, EDITED BY L. EGGELING AND M. BOTT, CRC PRESS

### Summary of the Invention

### Problems to be Solved by the Invention

An object of the present invention is to improve the production efficiency of at least one amino acid selected from the group consisting of L-arginine, L-citrulline, and L-ornithine by reducing or losing the activity to uptake amino acid in a coryneform bacterium.

### Means for Solving the Problems

The present invention relates to the following (1) to (4).
(1) A process for producing at least one amino acid selected from the group consisting of L-arginine, L-citrulline, and L-ornithine, comprising: culturing, in a medium, a coryneform bacterium in which the activity of uptake of the amino acid is reduced or lost as compared with a parent strain by introducing a deletion, a substitution, or an addition of at least one nucleotide into at least one gene which is present in the chromosomal DNA of the parent strain and encodes a protein selected from the group consisting of the following [1] to [6], and the coryneform bacterium can produce the amino acid; allowing the amino acid to be produced and accumulated in a culture; and collecting the amino acid from the culture,
   [1] a protein comprising the amino acid sequence represented by SEQ ID NO:2,
   [2] a protein comprising the amino acid sequence represented by SEQ ID NO:3,
   [3] a protein comprising the amino acid sequence represented by SEQ ID NO:4,
   [4] a protein which consists of an amino acid sequence having 80% or more identity to the amino acid sequence represented by SEQ ID NO:2, and has the activity of uptake of the amino acid,
   [5] a protein which consists of an amino acid sequence having 80% or more identity to the amino acid sequence represented by SEQ ID NO:3, and has the activity of uptake of the amino acid, and
   [6] a protein which consists of an amino acid sequence having 80% or more identity to the amino acid sequence represented by SEQ ID NO:4, and has the activity of uptake of the amino acid.
(2) The process of (1) above, wherein the coryneform bacterium, in which the activity of uptake of at least one amino acid selected from the group consisting of L-arginine, L-citrulline, and L-ornithine is reduced or lost as compared with a parent strain by introducing a deletion, a substitution, or an addition of at least one nucleotide into at least one gene which is present in the chromosomal DNA of the parent strain and encodes a protein selected from the group consisting of [1] to [6] in (1) above, and the coryneform bacterium can produce the amino acid, is a coryneform bacterium in which a deletion, a substitution, or an addition of at least one nucleotide is introduced into a DNA which is present in the chromosomal DNA of the parent strain and is selected from the group consisting of the following [7] to [9],
   [7] a DNA comprising the nucleotide sequence represented by SEQ ID NO: 1,
   [8] a DNA which hybridizes with a DNA consisting of a nucleotide sequence complementary to the nucleotide sequence represented by SEQ ID NO:1 under stringent conditions, and encodes a protein having the activity of uptake of the amino acid, and
   [9] a DNA which consists of a nucleotide sequence having 80% or more identity to the nucleotide sequence represented by SEQ ID NO: 1, and encodes a protein having the activity of uptake of the amino acid.
(3) The process of (1) or (2) above, wherein the coryneform bacterium is Corynebacterium glutamicum.
(4) The process of any one of (1) to (3) above, wherein the amino acid is L-arginine or L-citrulline.

### Effects of the Invention

According to the present invention, a process for producing at least one amino acid selected from the group consisting of L-arginine, L-citrulline, and L-ornithine using a coryneform bacterium, in which the activity of uptake of the amino acid is reduced or lost as compared with that of a parent strain, whereby the productivity of the amino acid is improved is provided.

### Embodiments for Carrying Out the Invention

### 1. Coryneform bacterium to be used in the present invention

The coryneform bacterium to be used in the present invention is a coryneform bacterium in which the activity of uptake of at least one amino acid selected from the group consisting of L-arginine, L-citrulline, and L-ornithine is reduced or lost as compared with that of a parent strain by introducing a deletion, a substitution, or an addition of at least one nucleotide into at least one gene which is present in the chromosomal DNA of the parent strain and encodes a protein selected from the group consisting of the following [1] to [6], and the coryneform bacterium can produce the amino acid:
[1] a protein comprising the amino acid sequence represented by SEQ ID NO:2,
[2] a protein comprising the amino acid sequence represented by SEQ ID NO:3,
[3] a protein comprising the amino acid sequence represented by SEQ ID NO:4,
[4] a protein which consists of an amino acid sequence having 80% or more identity to the amino acid sequence represented by SEQ ID NO:2, and has the activity of uptake of the amino acid,
[5] a protein which consists of an amino acid sequence having 80% or more identity to the amino acid sequence represented by SEQ ID NO:3, and has the activity of uptake of the amino acid, and
[6] a protein which consists of an amino acid sequence having 80% or more identity to the amino acid sequence represented by SEQ ID NO:4, and has the activity of uptake of the amino acid.

Further, examples of the coryneform bacterium to be used in the present invention may include a coryneform bacterium in which the activity of uptake of the amino acid is reduced or lost as compared with that of a parent strain by introducing a deletion, a substitution, or an addition of at least one nucleotide into at least one gene which is present in the chromosomal DNA of the parent strain and is selected from the group consisting of the following [7] to [9], and the coryneform bacterium can produce the amino acid:
[7] a DNA comprising the nucleotide sequence represented by SEQ ID NO:1,
[8] a DNA which hybridizes with a DNA consisting of a nucleotide sequence complementary to the nucleotide sequence represented by SEQ ID NO:1 under stringent conditions, and encodes a protein having the activity of uptake of the amino acid, and
[9] a DNA which consists of a nucleotide sequence having 80% or more identity to the nucleotide sequence represented by SEQ ID NO: 1, and encodes a protein having the activity of uptake of the amino acid.

The proteins consisting of the amino acid sequences represented by SEQ ID NOS:2, 3, and 4, respectively, are encoded by DNAs consisting of the nucleotide sequences represented by 301 to 1164, 1312 to 2157, and 2173 to 2925 of SEQ ID NO:1, respectively.

The gene as used herein refers to a DNA which may contain a transcriptional regulatory region, a promoter region, and the like in addition to a coding region of a protein.

The transcriptional regulatory region may include a DNA consisting of 100 nucleotides, preferably 50 nucleotides upstream of the 5' end of the coding region in a chromosomal DNA. The promoter region may include a region corresponding to -10 and -35 region.

Examples of the coryneform bacterium, which can produce at least one amino acid selected from the group consisting of L-arginine, L-citrulline, and L-ornithine, and in which the activity of uptake of the amino acid is reduced or lost as compared with that of a parent strain, and which can produce the amino acid, may include a coryneform bacterium which is obtained by introducing a deletion, a substitution, or an addition of at least one nucleotide into at least one gene, which is present in the chromosomal DNA of the parent strain and encodes a protein selected from the group consisting of the above-described [1] to [6], or a DNA, which is present in the chromosomal DNA of the parent strain and is selected from the group consisting of the above-described [7] to [9], and in which (a) the specific activity of a protein encoded by the gene or the DNA is reduced to 80% or less, preferably 50% or less, more preferably 30% or less, further more preferably 20% or less, particularly preferably 10% or less, and most preferably 0% as compared with that of the parent strain; or (b) the transcription amount of the gene or the DNA or the production amount of a protein encoded by the gene or the DNA is reduced to 80% or less, preferably 50% or less, more preferably 30% or less, further more preferably 20% or less, particularly preferably 10% or less, and most preferably 0% as compared with that of the parent strain. More preferable examples thereof may include a coryneform bacterium, in which the gene or the DNA is partially or completely deleted.

In the introduction of a deletion, a substitution, or an addition of at least one nucleotide into at least one gene, which is present in the chromosomal DNA of the parent strain and encodes a protein selected from the group consisting of the above-described [1] to [6], or a DNA, which is present in the chromosomal DNA of the parent strain and is selected from the group consisting of the above-described [7] to [9], the type of the nucleoide and the number of the nucleotides are not limited as long as the deletion, substitution, or addition of at least one nucleotide causes reduction or loss of the activity of uptake of a protein encoded by the gene or the DNA as compared with that of the parent strain. The deletion of a nucleotide may include, in the case of a promoter or a transcriptional regulatory region, a deletion of 1 nucleotide or more, preferably 10 nucleotides or more, more preferably 20 nucleotides or more, and further more preferably the whole of the region, and in the case of a coding region, a deletion of 1 nucleotide or more, preferably 10 nucleotides or more, more preferably 20 nucleotides or more, further more preferably 100 nucleotides or more, particularly preferably 200 nucleotides or more, and most preferably the whole of the coding region.

The substitution of at least one nucleotide may include, a substitution of a nucleotide within the 150 nucleotides, preferably a nucleotide within the 100 nucleotides, more preferably a nucleotide within the 50 nucleotides, particularly preferably a nucleotide within the 30 nucleotides, and most preferably a nucleotide within the 20 nucleotides from the 5' end of a coding region to introduce a nonsense codon.

The addition of at least one nucleotide may include, an addition of a DNA fragment of 1 nucleotide or more, preferably 50 nucleotides or more, more preferably 100 nucleotides or more, further more preferably 200 nucleotides or more, particularly preferably 500 nucleotides or more, and most preferably 1 kb or more to a site immediately downstream of a nucleotide within the 150 nucleotides, preferably a nucleotide within the 100 nucleotides, more preferably a nucleotide within the 50 nucleotides, particularly preferably a nucleotide within the 30 nucleotides, and most preferably a nucleotide within the 20 nucleotides from the 5' end of the coding region. Most preferable examples may include an insertion of a chloramphenicol resistance gene, a kanamycin resistance gene, or the like.

The "activity of uptake of at least one amino acid selected from the group consisting of L-arginine, L-citrulline, and L-ornithine" as used herein refers to the activity of uptake of at least one amino acid selected from the group consisting of L-arginine, L-citrulline, and L-ornithine, preferably L-arginine or L-citrulline, more preferably L-arginine into the cell from outside of the cell of the coryneform bacterium.

Whether or not the activity of uptake of the amino acid is reduced as compared with that of a parent strain can be confirmed by determining the transcription amount of at least one gene encoding a protein selected from the group consisting of the above-described [1] to [6], or a DNA selected from the group consisting of the above-described [7] to [9] by a Northern analysis or RT-PCR, and comparing the transcription amount with that of the parent strain, or determining the production amount of a protein encoded by the gene or the DNA by SDS-PAGE or an assay using an antibody, and comparing the production amount with that of the parent strain, and so on.

Further, it can be confirmed that the activity of uptake of the amino acid is reduced or lost as compared with that of a parent strain by confirming that when the bacterium is cultured on an agar medium containing the amino acid as a sole carbon source, the diameter of a colony after a predetermined period of time is smaller than that of the parent strain or the bacterium does not grow.

The identity of amino acid sequences or nucleotide sequences can be determined using the algorithm BLAST by Karlin and Altschul (PROCEEDINGS OF THE NATIONAL ACADEMY OF SCIENCES, 1993, vol. 90, No. 12, pp. 5873-5877) or FASTA (METHODS IN ENZYMOLOGY, 1990, vol. 183, pp. 63-98). Based on the algorithm BLAST, programs called BLASTN and BLASTX have been developed (JOURNAL OF MOLECULAR BIOLOGY, 1990, vol. 215, pp. 403-410). In the case where a nucleotide sequence is analyzed using BLASTN based on BLAST, the parameters are set, for example, as follows: score = 100 and word length = 12. In the case where an amino acid sequence is analyzed using BLASTX based on BLAST, the parameters are set, for example, as follows: score = 50 and word length = 3. In the case where BLAST and Gapped BLAST programs are used, default parameters for each of the programs are employed. The specific technique for these analytical methods is well known.

The "hybridization" as used above refers to hybridization of a DNA with a DNA comprising a specific nucleotide sequence or a part of the DNA under specific conditions. Therefore, the nucleotide sequence of the DNA comprising a specific nucleotide sequence or a part of the DNA may be a DNA which is useful as a probe in a Northern or Southern blot analysis, or may be a DNA having a length which can be used as an oligonucleotide primer in a PCR analysis. The DNA to be used as a probe may include a DNA of at least 100 nucleotides or more, preferably 200 nucleotides or more, and more preferably 500 nucleotides or more, but a DNA of at least 10 nucleotides or more, and preferably 15 nucleotides or more may also be included.

A method for DNA hybridization experiment is well known, and the experiment can be performed by, for example, determining the conditions for hybridization according to the description in MOLECULAR CLONING, 2nd and 3rd Ed. (2001), METHODS FOR GENERAL AND MOLECULAR BACTERIOLOGY, ASM PRESS (1994), or IMMUNOLOGY METHODS MANUAL, ACADEMIC PRESS (MOLECULAR), and also, according to any of a number of other standard textbooks.

Further, also according to an instructional manual accompanying a commercially available hybridization kit, a DNA which hybridizes under stringent conditions can be obtained. The commercially available hybridization kit may include, for example, Random Primed DNA Labeling Kit (manufactured by Roche Diagnostics GmbH), with which a probe is produced by a random prime method, and hybridization is performed under stringent conditions, and the like.

The above-described stringent conditions may include conditions in which a filter on which a DNA has been immobilized and a probe DNA are incubated overnight at 42°C in a solution containing 50% formamide, 5 x SSC (750 mM sodium chloride and 75 mM sodium citrate), 50 mM sodium phosphate (pH 7.6), 5 x Denhardt's solution, 10% dextran sulfate, and 20 µg/l of a denatured salmon sperm DNA, and then the filter is washed in, for example, a 0.2 x SSC solution at about 65°C.

The above-described various conditions can also be set by adding or changing a blocking reagent to be used for suppressing the background in the hybridization experiment. The addition of the blocking reagent may be accompanied by a change in hybridization conditions for adapting the conditions.

The DNA which can hybridize under the above-described stringent conditions may include a DNA consisting of a nucleotide sequence having at least 80% or more, preferably 90% or more, more preferably 95% or more, further more preferably 98% or more, and particularly preferably 99% or more identity to the nucleotide sequence represented by SEQ ID NO:1 when performing calculation based on the above-described parameters using, for example, the program such as BLAST or FASTA described above.

The parent strain as used herein refers to an original strain to be subjected to genetic engineering, transformation, or the like. The original strain to be subjected to genetic transformation by gene transfer is also referred to as a host strain.

The expression "can produce the amino acid" refers to possession of an ability to produce the amino acid to such an extent that the amino acid can be collected from the cells or the medium when the coryneform bacterium to be used in the present invention is cultured in the medium.

The coryneform bacterium which can produce the amino acid may include, in the case where a parent strain originally has a property capable of producing the amino acid, a coryneform bacterium in which the property has been enhanced, and in the case where a parent strain does not have the property, a coryneform bacterium to which the property has been artificially imparted.

The coryneform bacterium to be used in the present invention may include a coryneform bacterium belonging to the genus Corynebacterium, the genus Brevibacterium, or the genus Microbacterium.

Examples of a microorganism belonging to the genus Corynebacterium may include Corynebacterium glutamicum, Corynebacterium acetoacidophilum, Corynebacterium acetoglutamicum, Corynebacterium callunae, Corynebacterium herculis, Corynebacterium lilium, Corynebacterium melassecola, Corynebacterium thermoaminogenes, and the like, and specific examples thereof may include Corynebacterium glutamicum ATCC 13032, Corynebacterium glutamicum ATCC 13060, Corynebacterium glutamicum ATCC 13826 (former name: Brevibacterium flavum), Corynebacterium glutamicum ATCC 14020 (former name: Brevibacterium divaricatum), Corynebacterium glutamicum ATCC 13869 (former name: Brevibacterium lactofermentum), Corynebacterium acetoacidophilum ATCC 13870, Corynebacterium acetoglutamicum ATCC 15806, Corynebacterium callunae ATCC 15991, Corynebacterium herculis ATCC 13868, Corynebacterium lilium ATCC 15990, Corynebacterium melassecola ATCC 17965, Corynebacterium thermoaminogenes ATCC 9244, and the like.

Examples of a microorganism belonging to the genus Brevibacterium may include Brevibacterium saccharolyticum, Brevibacterium immariophilum, Brevibacterium roseum, Brevibacterium thiogenitalis, and the like, and specific examples thereof may include Brevibacterium saccharolyticum ATCC 14066, Brevibacterium immariophilum ATCC 14068, Brevibacterium roseum ATCC 13825, Brevibacterium thiogenitalis ATCC 19240, and the like.

Examples of a microorganism belonging to the genus Microbacterium may include Microbacterium ammoniaphilum and the like, and specific examples thereof may include Microbacterium ammoniaphilum ATCC 15354 and the like.

### 2. Process for preparing coryneform bacterium to be used in the Invention

The coryneform bacterium to be used in the present invention can be prepared by introducing a deletion, a substitution, or an addition of at least one nucleotide into at least one gene, which is present in the chromosomal DNA of the parent strain and encodes a protein selected from the group consisting of the above-described [1] to [6], or a DNA, which is present in the chromosomal DNA of the parent strain and is selected from the group consisting of the above-described [7] to [9], and selecting a coryneform bacterium in which the production amount of at least one amino acid selected from the group consisting of L-arginine, L-citrulline, and L-ornithine is improved as compared with that of the parent strain.

The introduction of a deletion, a substitution, or an addition of at least one nucleotide into a gene, which is present in the chromosomal DNA of the parent strain is not limited as long as it is a method capable of introducing a mutation into the chromosomal DNA of a coryneform bacterium such as a usual mutation treatment method, a gene substitution method by a recombinant DNA technique or the like, a cell fusion method, or a transduction method.

The parent strain may be a wild-type strain or a breeding strain which has been artificially bred from the wild-type strain as long as it is a coryneform bacterium which has an ability to produce the amino acid and also has the activity of uptake of the amino acid.

Examples of the method for artificially imparting an ability to produce the amino acid to a coryneform bacterium may include:
(a) a method of relieving or canceling at least one mechanism of controlling the biosynthesis of the amino acid;
(b) a method of enhancing the expression of at least one enzyme involved in the biosynthesis of the amino acid;
(c) a method of increasing the number of copies of at least one gene encoding an enzyme involved in the biosynthesis of the amino acid;
(d) a method of attenuating or blocking at least one metabolic pathway branching from the biosynthetic pathway of the amino acid into a metabolite other than the target substance; and
(e) a method of selecting a cell line having a higher degree of resistance to an analog of the amino acid as compared with the wild-type strain.

The above described known methods can be used alone or in combination with one another.

As a method for preparing a coryneform bacterium which has an ability to produce the amino acid using any of the above-described methods (a) to (e) or a method in combination thereof, a lot of examples are described in Biotechnology 2nd ed., Vol. 6, Products of Primary Metabolism (VCH Verlagsgesellschaft mbH, Weinheim, 1996) section 14a, 14b or Advances in Biochemical Engineering/ Biotechnology, 79, 1-35 (2003), Agric. Biol. Chem., 51, 2089-2094 (1987), and Amino Acid Fermentation, Gakkai Shuppan Center, Hiroshi Aida, et al., (1986). Further, other than the above-described publications, a specific method for preparing a coryneform bacterium which has an ability to produce an amino acid has been reported in a lot of publications such as JP-A-2003-164297, Agric. Biol. Chem., 39, 153-160 (1975), Agric. Biol. Chem., 39, 1149-1153 (1975), JP-A-58-13599, J. Gen. Appl. Microbiol., 4, 272-283 (1958), JP-A-63-94985, Agric. Biol. Chem., 37, 2013-2023 (1973), WO 97/15673, JP-A-56-18596, JP-A-56-144092, JP-T-2003-511086, and WO 2006/001380, and the coryneform bacterium which has an ability to produce the amino acid can be prepared with reference to any of the above-described publications, or the like.

Examples of the coryneform bacterium which has an ability to produce L-arginine and can be prepared by the above-described method may include Corynebacterium glutamicum to which D-arginine resistance and arginine hydroxamate resistance have been imparted (AGRICULTURAL AND BIOLOGICAL CHEMISTRY, 1972, vol. 36, No. 10, pp. 1675-1684) and Corynebacterium glutamicum into which a mutation to delete the argR gene has been introduced (JP-A-2002-51790).

Examples of the coryneform bacterium which has an ability to produce L-ornithine, L-citrulline, or L-arginine include Corynebacterium glutamicum in which the productivity of L-ornithine, L-citrulline, or L-arginine is improved by introducing a mutation to delete the argR gene so as to cause disinhibition of the arg operon and also introducing a mutation in the argB gene so as to cause desensitization to an acetylglutamate kinase (W2006/035831 and APPLIED AND ENVIRONMENTAL MICROBIOLOGY, 2009, vol. 75, No. 6, pp. 1635-1641).

A coryneform bacterium which can be used for preparing the above-described coryneform bacterium having an ability to produce the amino acid may be any bacterium as long as it is a coryneform bacterium to which any of the above-described methods (a) to (e) can be applied or a coryneform bacterium which has the above-described genetic characteristics, and preferable examples thereof may include the above-described coryneform bacteria belonging to the genus Corynebacterium, the genus Brevibacterium, or the genus Microbacterium.

Examples of the mutation treatment method may include a method using N-methyl-N'-nitro-N-nitrosoguanidine (NTG) (Microorganism Experiment Manual, 1986, p. 131, Kodansha Scientific, Ltd.), a UV irradiation method, and the like.

Examples of the gene substitution method in which a substitution, a deletion, or an addition of at least one nucleotide is introduced into at least one gene encoding a protein selected from the group consisting of the above-described [1] to [6], or a DNA selected from the group consisting of the above-described [7] to [9] by a recombinant DNA technique may include a method in which the gene is integrated into the chromosome of a parent strain by homologous recombination or the like, and further, the gene originally present in the chromosome is substituted by homologous recombination or the like.

Examples of the method for introducing a substitution, a deletion, or an addition of at least one nucleotide into the gene may include, other than the above-described methods, a method in accordance with a site-specific mutagenesis method described in Molecular cloning: a laboratory manual, 3rd ed., Cold Spring Harbor Laboratory Press (2001) (hereinafter abbreviated as Molecular cloning 3rd ed.), Current Protocols in Molecular Biology, John Wiley & Sons (1987-1997) (hereinafter abbreviated as Current Protocols in Molecular Biology), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985), or the like.

The gene substitution by a recombinant DNA technique can be performed according to the method described in J. Bacteriol., 182, 6884 (2000) or the like.

By inserting a gene into which a mutation has been introduced into an appropriate plasmid vector or the like, a recombinant plasmid is produced. As the plasmid vector, for example, a plasmid which cannot autonomously replicate in the parent strain, and has an antibiotic resistance marker gene and a levan sucrase gene sacB of Bacillus subtilis [Mol. Microbiol., 6, 1195 (1992)] can be used.

As the method for introducing the recombinant plasmid into the parent strain, any method can be used as long as it is a method capable of introducing a DNA into a coryneform bacterium, and examples thereof may include an electroporation method [Appl. Microbiol. Biotech., 52, 541 (1999)], a protoplast method [J. Bacteriol., 159, 306 (1984)], and the like.

Since the recombinant plasmid cannot autonomously replicate in the parent strain, by obtaining a strain which shows resistance to an antibiotic corresponding to the antibiotic resistance marker contained in the recombinant plasmid, a transformant strain in which the recombinant plasmid has been integrated into the chromosome can be obtained.

Further, by a selection method utilizing the fact that the levan sucrase of Bacillus subtilis integrated in the chromosome along with the gene into which the mutation has been introduced produces a suicide substrate [J. Bacteriol., 174, 5462 (1992)], a strain in which the gene in the chromosome of the parent strain has been substituted with the gene into which the mutation has been introduced can be obtained.

By the above-described method, the substitution of a gene in the chromosome of a parent strain can be performed, however, it is not limited to the above-described method, and another gene substitution method can also be used as long as it is a method capable of substituting a gene in the chromosome of a coryneform bacterium.

Examples of the method for introducing a substitution, a deletion, or an addition into a gene in the chromosome of a parent strain may include a cell fusion method and a transduction method other than the above-described methods, and may include, for example, the method described in Amino Acid Fermentation, Gakkai Shuppan Center, edited by Hiroshi Aida, et al., (1986) and the like.

The number of nucleotides and the site for introducing a mutation are as described in the above item 1.

By introducing a deletion, a substitution, or an addition of at least one nucleotide into a gene in the chromosome of a parent strain, the activity of a protein encoded by the gene can be reduced or lost with high probability (An Introduction to Genetic Analysis, 7th edition, Griffiths AJF, Miller JH, Suzuki DT, et al. New York, W. H. Freeman, 2000).

Each of the genes encoding the above-described proteins [1] to [6] can be obtained by PCR using, for example, a chromosomal DNA prepared according to the method of Saito et al. (BIOCHIMICA ET BIOPHYSICAACTA, 1963, vol. 72, pp. 619-629) from a microorganism belonging to the coryneform bacteria as a template, and also using, as a primer, an oligo DNA designed and synthesized based on the nucleotide sequence represented by 2173 to 2925 of SEQ ID NO:1, 1312 to 2157 of SEQ ID NO: 1, or 301 to 1164 of SEQ ID NO:1.

A specific gene which can be obtained may include NCg11276 comprising the nucleotide sequence represented by 2173 to 2925 of SEQ ID NO: 1, NCgl1277 comprising the nucleotide sequence represented by 1312 to 2157 of SEQ ID NO:1, NCgl1278 comprising the nucleotide sequence represented by 301 to 1164 of SEQ ID NO:1, and the like.

Incidentally, in the case where at least two genes are adjacent in the chromosome or contained in the operon DNA, the at least two genes can also be obtained, for example, as one DNA by PCR using, as a primer, an oligo DNA designed and synthesized based on the nucleotide sequence represented by SEQ ID NO: 1.

Further, the gene can also be obtained by a hybridization method using a part or the whole of the above-described DNA as a probe, a method in which a DNA comprising the above-described nucleotide sequence is chemically synthesized according to a known method, or the like.

Further, the gene can also be obtained by searching, through various gene sequence databases, for a sequence having 85% or more, preferably 90% or more, more preferably 95% or more, further more preferably 98% or more, and particularly preferably 99% or more homology or identity to the nucleotide sequence of a DNA encoding the amino acid sequence represented by SEQ ID NO:2, 3, or 4, and obtaining the desired gene based on the nucleotide sequence obtained by the search, from a chromosomal DNA, cDNA library, or the like of an organism having the nucleotide sequence according to the above-described method.

The nucleotide sequence of a DNA can be determined by a conventionally used nucleotide sequence analysis method, such as a dideoxy method (PROCEEDINGS OF THE NATIONAL ACADEMY OF SCIENCES, 1977, vol. 74, No. 12, pp. 5463-5467), or by performing an analysis using a nucleotide sequence analyzer such as the 373A DNA Sequencer (manufactured by PerkinElmer Co., Ltd.).

In the case where the obtained DNA is a partial-length DNA as a result of determination of the nucleotide sequence, a full-length DNA can be obtained by a Southern hybridization method or the like for a chromosomal DNA library using the partial-length DNA as a probe.

The measurement of the activity of amino acid uptake into the cell can be performed according to the method described in THE JOURNAL OF BIOLOGICAL CHEMISTRY, 1971, vol. 246, No. 11, pp. 3653-3662.

According to the above-described method, the coryneform bacterium to be used in the process of the present invention can be prepared.

### 3. Process for producing L-arginine, L-citrulline, and L-ornithine of the present invention

A coryneform bacterium which can be prepared by the above-described method is cultured in a medium, and allowed to produce and accumulate at least one amino acid selected from the group consisting of L-arginine, L-citrulline, and L-ornithine, and the amino acid is collected from the culture, whereby the amino acid can be produced.

As the medium to be used in the production process of the present invention, either a synthetic medium or a natural medium may be used as long as it contains nutrients necessary for the growth of the microorganism of the present invention and the biosynthesis of an amino acid selected from the group consisting of L-arginine, L-citrulline, and L-ornithine such as a carbon source, a nitrogen source, and inorganic salts.

As the carbon source, any carbon source may be used as long as it can be assimilated by the microorganism to be used, and examples thereof may include sugars such as glucose, molasses, fructose, sucrose, maltose, and a starch hydrolysate, alcohols such as ethanol and glycerol, organic acids such as acetic acid, lactic acid, and succinic acid, and the like.

As the nitrogen source, ammonia, a variety of inorganic and organic ammonium salts such as ammonium chloride, ammonium sulfate, ammonium carbonate, and ammonium acetate, nitrogen compounds such as urea and amine, and nitrogen-containing organic substances such as meat extract, yeast extract, corn steep liquor, peptone, and a soybean hydrolysate, can be used.

As the inorganic salts, potassium monohydrogen phosphate, potassium dihydrogen phosphate, ammonium sulfate, magnesium sulfate, sodium chloride, ferrous sulfate, calcium carbonate, and the like can be used.

Other than these, a minor nutrient source such as biotin, thiamine, nicotinamide, or nicotinic acid can be added as needed. Such a minor nutrient source can also be substituted with a medium additive such as meat extract, corn steep liquor, casamino acid or the like. Further, a substance required for the microorganism of the present invention to grow (for example, a required amino acid in the case of an amino acid auxotrophic microorganism) can be added as needed.

The culturing is performed under aerobic conditions such as shaking culture or submerged spinner culture under aeration. The culturing temperature is from 20 to 50°C, preferably from 20 to 42°C, and more preferably from 28 to 38°C. The culturing is performed by maintaining the pH of the medium in the range from 5 to 11, preferably in the range from 6 to 9 around a neutral pH. The pH of the medium is adjusted with an inorganic or organic acid, an alkaline solution, urea, calcium carbonate, ammonia, a pH buffer, or the like.

The culturing period is from 5 hours to 6 days, preferably from 16 hours to 3 days.

The amino acid accumulated in the culture can be collected by a conventional purification method. For example, L-arginine can be collected by removing the bacterial cells and solid substances by centrifugation or the like after culturing, and then combining known methods such as active carbon treatment, ion-exchange resin treatment, condensation, and crystallization separation.

Hereinafter, Examples of the invention of this application will be described, however, the present invention is not limited to these Examples.

### Example 1

### (1) Construction of plasmid for gene disruption

A plasmid pHSG299 (GENE, 1987, vol. 61, pp. 63-74) having a gene imparting kanamycin resistance was digested with a restriction enzyme PstI. Then, a DNA fragment of 2.6 kilobases (hereinafter abbreviated as kb) containing a levan sucrase gene sacB derived from a Bacillus subtilis 168 strain (MOLECULAR MICROBIOLOGY, 1992, vol. 6, No. 9, pp. 1195-1202) was ligated to the plasmid at the cleavage site, whereby a plasmid pESB30 was obtained.

pESB30 was digested with a restriction enzyme BamHI, followed by extraction and purification. Thereafter, both ends of the obtained DNA fragment were blunted using a DNA blunting kit (manufactured by Takara Bio, Inc.) according to the accompanying protocol. The blunted DNA fragment was reacted at 70°C for 2 hours in the presence of Taq polymerase (manufactured by Boehringer Mannheim GmbH) and dTTP so that one thymine base was added to the 3' end, whereby a DNA fragment pESB30-T which can be used for PCR fragment cloning was prepared.

### (2) Construction of plasmid for creating argF gene disruption strain

By using the chromosomal DNA of a Corynebacterium glutamicum ATCC 13032 strain as a template, and also using a DNA consisting of the nucleotide sequence represented by SEQ ID NO:5 and a DNA consisting of the nucleotide sequence represented by SEQ ID NO:6 as a primer set, PCR was performed using Pfu turbo DNA polymerase (manufactured by Stratagene Corporation) and the accompanying buffer. A DNA fragment of about 1.4 kb obtained by the PCR was digested with BamHI, followed by extraction and purification.

Thus obtained DNA fragment was mixed with pUC119 (manufactured by Takara Bio, Inc.) previously digested with BamHI, and a DNA ligase reaction was performed using a DNA ligation kit (manufactured by Takara Bio, Inc.). By using the reaction product, an Escherichia coli DH5α strain (manufactured by Toyobo Co., Ltd.) was transformed according to the method described in Molecular cloning 3rd ed.

From the transformant, a plasmid was prepared according to the alkali SDS method (the method described in Molecular cloning 3rd ed.). Thus prepared plasmid was digested with NcoI, and then self-circularized by a DNA ligase reaction. Thereafter, an Escherichia coli DH5α strain (manufactured by Toyobo Co., Ltd.) was transformed, and the plasmid was prepared in the same manner as described above.

The structure of the plasmid was examined by digestion patterns with several types of restriction enzymes, and it was confirmed that the plasmid contains a DNA fragment in which 369 base pairs were deleted in the DNA encoding argF. By using the plasmid as a template, and also using a DNA consisting of the nucleotide sequence represented by SEQ ID NO:5 and a DNA consisting of the nucleotide sequence represented by SEQ ID NO:6 as a primer set, PCR was performed, whereby a DNA fragment of about 1.0 kb was obtained. The DNA fragment was reacted at 72°C for 10 minutes in the presence of Taq DNA polymerase (manufactured by Boehringer Mannheim GmbH) and dATP, whereby one adenine base was added to the 3' end.

Thus obtained DNA fragment and the previously created pESB30-T were mixed, and a DNA ligase reaction was performed. By using the reaction product, an Escherichia coli DH5α strain (manufactured by Toyobo Co., Ltd.) was transformed, and the plasmid was prepared from the obtained transformant. The plasmid was designated as pEargF.

### (3) Preparation of argF gene disruption strain

By using the plasmid pEargF prepared as described above, a transposon-disrupted Corynebacterium glutamicum ATCC 13032 strain (hereinafter referred to as a DOI-3 strain) was transformed by the electroporation method according to the method of Rest et al.(APPLLIED MICROBIOLOGY AND BIOTECHNOLOGY, 1999, vol. 52, No. 4, pp. 541-545), and a kanamycin resistant strain was selected.

The chromosomal DNA of the kanamycin resistant strain was prepared and examined by Southern hybridization (Molecular cloning 3rd ed.), and as a result, it was confirmed that pEargF was integrated by Campbell-type homologous recombination.

In such a strain, a region including the argF gene originally present in the chromosome and a region having a structure in which the argF gene in pEargF has been deleted are present in proximity to each other, and the second homologous recombination is easy to occur therebetween.

Since levan sucrose encoded by the sacB gene converts sucrose into a suicide substrate, a microorganism having the sacB gene cannot grow in a medium containing sucrose. However, in a strain in which the second homologous recombination has occurred between a region including the argF gene originally present in the chromosome and a region having a structure in which the argF gene in pEargF has been deleted, either DNA region is deleted along with sacB, and therefore, the strain can grow even in a medium containing sucrose. In this manner, a microorganism having a structure in which the argF gene originally present in the chromosomal DNA is deleted can be obtained.

By utilizing this, the above-described transformant strain was applied on a sucrose agar medium [a medium containing 100 g of sucrose, 7 g of meat extract, 10 g of peptone, 3 g of sodium chloride, 5 g of yeast extract (manufactured by Difco Co., Ltd.), and 15 g of Bacto agar (manufactured by Difco Co., Ltd.) in 1 L of water, and adjusted to pH 7.2], and cultured at 30°C for 1 day, and then, a growing colony was selected. The strain was designated as DOI-3argF strain.

### (4) Confirmation of auxotrophy of argF gene disruption strain

The DOI-3argF strain contains a deletion in the argF gene, and therefore exhibits auxotrophy for L-citrulline and L-arginine. This was confirmed by the fact that the DOI-3argF strain did not grow in a minimal medium, but grew in a medium obtained by adding 50 mg/L L-arginine or 50 mg/L L-citrulline to the minimal medium.

### Example 2

### (1) Construction of plasmid for creating NCgl1278 gene disruption strain

By using the chromosomal DNA of a Corynebacterium glutamicum ATCC 13032 strain as a template, and also using a DNA consisting of the nucleotide sequence represented by SEQ ID NO:7 and a DNA consisting of the nucleotide sequence represented by SEQ ID NO:8 as a primer set for a reaction 1, and using a DNA consisting of the nucleotide sequence represented by SEQ ID NO:9 and a DNA consisting of the nucleotide sequence represented by SEQ ID NO:10 as a primer set for a reaction 2, PCR for the reaction 1 and PCR for the reaction 2 were performed separately.

A DNA fragment of about 1.0 kb obtained by the PCR was extracted and purified. By using the DNA fragment obtained by the reaction 1 and the DNA fragment obtained by the reaction 2 as template DNAs, PCR was performed using a DNA consisting of the nucleotide sequence represented by SEQ ID NO:7 and a DNA consisting of the nucleotide sequence represented by SEQ ID NO:10 as a primer set for a reaction 3. A DNA fragment of about 1.0 kb obtained by the PCR was purified, digested with a restriction enzyme FbaI, and purified again.

Thus obtained DNA fragment was mixed with pESB30 which was previously digested with a restriction enzyme BamHI and was dephosphorylated with alkaline phosphatase (manufactured by Takara Bio, Inc.), and a DNA ligase reaction was performed. By using the reaction product, an Escherichia coli DH5α strain (manufactured by Toyobo Co., Ltd.) was transformed, and the plasmid was prepared from the transformant strain. The plasmid was designated as pdel1278.

### (2) Preparation of argF and NCgl1278 gene disruption strain

By using the thus prepared plasmid pdel1278, the DOI-3argF strain was transformed by the electroporation method in the same manner as in Example 1, whereby a kanamycin resistant strain was obtained. Thereafter, by using the same method as described in Example 1, a strain in which the ORF of NCgl1278 was deleted was created, and named DOI-3argF_Del1278 strain.

### (3) Confirmation of activity of L-citrulline and L-arginine uptake of NCgl1278 gene product

As shown in Table 1, the DOI-3argF_Del1278 strain did not grow on a minimal medium agar plate containing 50 mg/L L-arginine or on a minimal medium agar plate containing 50 mg/L L-citrulline, but grew on a minimal medium agar plate containing 50 mg/L alanylarginine. Therefore, it was concluded that NCgl1278 is a gene encoding a protein involved in the uptake of L-arginine and L-citrulline into the cell.

Non-Patent Document 8 describes that NCgl1278 is classified in the ATP-binding Cassette (ABC) superfamily along with the following two genes immediately downstream of NCgl1278: NCgl1277 consisting of the nucleotide sequence represented by 1312 to 2157 of SEQ ID NO:1 and NCg11276 consisting of the nucleotide sequence represented by 2173 to 2925 of SEQ ID NO:1.

Based on this, it was indicated that the gene products of NCgl1276, NCgl1277, and NCgl1278 constitute transporters classified in the ATP-binding Cassette (ABC) superfamily and are involved in the activity of uptake of L-arginine and L-citrulline into the cell.

**[Table 1]**

| Amino acid or dipeptide added to minimal medium | DOI-3argF strain | DOI-3argF_Del1278 strain |
|---|---|---|
| No addition | B | B |
| L-arginine | A | B |
| L-citrulline | A | B |
| L-alanyl-L-arginine | A | A |

| | | |
|---|---|---|
| A: The strain grew, B: The strain did not grow | | |

### Example 3

### (1) Preparation of L-arginine-producing strain

A chromosomal DNA was prepared from an RB26 strain (W2006/035831), which is a Corynebacterium glutamicum L-arginine-producing strain, and by using the chromosomal DNA as a template, PCR was performed using a DNA consisting of the nucleotide sequence represented by SEQ ID NO: 11 and a DNA consisting of the nucleotide sequence represented by SEQ ID NO: 12 as a primer set, whereby a DNA fragment corresponding to a region having a length of about 3.7 kb containing argC, argJ, and argB genes was obtained. The RB26 strain has been revealed to include a mutation (A26V) in argB so that the feedback inhibitory effect of L-arginine is reduced (Non-patent Document 3).

The DNA fragment of about 3.7 kb was digested with restriction enzymes SalI and KpnI, followed by purification, and then, mixed with a plasmid pCS299P (APPLLIED MICROBIOLOGY AND BIOTECHNOLOGY, 2004, vol. 63, No. 5, pp. 592-601) which was previously digested with restriction enzymes SalI and KpnI, followed by purification, and a DNA ligase reaction was performed to effect ligation, whereby a plasmid pCS_argCJB26 was obtained.

In the same manner as the method for preparing the DOI-3argF_Del1278 strain in Example 2, a strain in which NCgl1278 was deleted was prepared using Corynebacterium glutamicum ATCC 13032 as a parent strain, and the prepared strain was designated as ATCC 13032_Del1278 strain.

By using the above plasmid pCS_argCJB26, the Corynebacterium glutamicum ATCC 13032 strain and the ATCC 13032_Del1278 strain were transformed by the electroporation method, and transformant strains which acquired kanamycin resistance were selected. These transformant strains were designated as 13032/pCS_argCJB26 strain and 13032_Del1278/pCS_argCJB26 strain, respectively.

### (2) Effect of deletion of NCgl1278 gene on production of L-arginine

Each of the ATCC 13032/pCS_argCJB26 strain and the ATCC 13032Del1278/pCS_argCJB26 strain was cultured at 30°C for 24 hours on a BY agar medium (a medium containing 7 g of meat extract, 10 g of peptone, 3 g of sodium chloride, 5 g of yeast extract, and 15 g of Bacto agar in 1 L of water, and adjusted to pH 7.2) containing 50 mg/L kanamycin.

The bacterial cells of each strain grown on the BY agar medium containing 50 mg/L kanamycin were inoculated in a thick test tube containing 6 ml of a seed medium (a medium containing 25 g of sucrose, 20 g of corn steep liquor, 20 g of peptone, 10 g of yeast extract, 0.5 g of magnesium sulfate heptahydrate, 2 g of potassium dihydrogen phosphate, 3 g of urea, 8 g of ammonium sulfate, 1 g of sodium chloride, 20 mg of nicotinic acid, 10 mg of iron sulfate heptahydrate, 10 mg of calcium pantothenate, 1 mg of zinc sulfate heptahydrate, 1 mg of copper sulfate pentahydrate, 1 mg of thiamine hydrochloride, and 100 µg of biotin in 1 L of water, and adjusted to pH 7.2 with an aqueous sodium hydroxide solution, followed by adding 10 g of calcium carbonate) containing 50 mg/L kanamycin, and cultured for 24 hours while shaking at 32°C.

2 ml of the seed culture broth obtained for each strain was inoculated in a flask with a baffle containing 20 ml of a main culture medium (a medium containing 60 g of glucose, 5 g of corn steep liquor, 30 g of ammonium sulfate, 8 g of potassium chloride, 2 g of urea, 0.5 g of potassium dihydrogen phosphate, 0.5 g of dipotassium hydrogen phosphate, 1 g of magnesium sulfate heptahydrate, 1 g of sodium chloride, 20 mg of iron sulfate heptahydrate, 20 mg of nicotinic acid, 20 mg of β-alanine, 10 mg of manganese sulfate pentahydrate, 10 mg of thiamine hydrochloride, and 200 µg of biotin in 1 L of water, and adjusted to pH 7.7 with an aqueous sodium hydroxide solution, followed by adding 30 g of calcium carbonate), and cultured for 48 hours while shaking at 32°C.

The bacterial cells were removed from the culture by centrifugation, and the accumulation amount of L-arginine in the supernatant was determined by high performance liquid chromatography (HPLC). The HPLC analysis was performed at 60°C using AQ-312 (manufactured by YMC America, Inc.) as a separation column, and a solution (pH 6.0) containing 2.94 g/L sodium citrate, 1.42 g/L sodium sulfate, 233 mL/L acetonitrile, and 3 g/L sodium lauryl sulfate as a mobile phase. The detection and determination of the amino acid were performed by mixing the eluate from the separation column with a reaction solution (a solution containing 18.5 g/L boric acid, 11 g/L NaOH, 0.6 g/L o-phthalaldehyde, 2 ml/L mercaptoethanol, and 3 mL/L Brige-35), and performing a fluorescence analysis at an excitation wavelength of 345 nm and an absorption wavelength of 455 nm. The results are shown in Table 2.

**[Table 2]**

| Strain | Accumulation amount of L-arginine (g/L) |
|---|---|
| ATCC 13032/pCS_argCJB26 strain | 1.1 ± 0.09 |
| ATCC 13032Del1278 strain/pCS_argCJB26 strain | 1.9 ± 0.22 |

### Industrial Applicability

According to the present invention, at least one amino acid selected from the group consisting of L-arginine, L-citrulline, and L-ornithine can be efficiently produced.

### Sequence Listing Free Text

SEQ ID NO:5 - Description of Artificial Sequence: Synthetic DNA
SEQ ID NO:6 - Description of Artificial Sequence: Synthetic DNA
SEQ ID NO:7 - Description of Artificial Sequence: Synthetic DNA
SEQ ID NO:8 - Description of Artificial Sequence: Synthetic DNA
SEQ ID NO:9 - Description of Artificial Sequence: Synthetic DNA
SEQ ID NO:10 - Description of Artificial Sequence: Synthetic DNA
SEQ ID NO:11 - Description of Artificial Sequence: Synthetic DNA
SEQ ID NO:12 - Description of Artificial Sequence: Synthetic DNA

## Claims

1. A process for producing at least one amino acid selected from the group consisting of L-arginine, L-citrulline, and L-ornithine, comprising: culturing, in a medium, a coryneform bacterium in which the activity of uptake of the amino acid is reduced or lost as compared with a parent strain by introducing a deletion, a substitution, or an addition of at least one nucleotide into at least one gene which is present in the chromosomal DNA of the parent strain and encodes a protein selected from the group consisting of the following [1] to [6], and the coryneform bacterium can produce the amino acid; allowing the amino acid to be produced and accumulated in a culture; and collecting the amino acid from the culture,
[1] a protein comprising the amino acid sequence represented by SEQ ID NO:2,
[2] a protein comprising the amino acid sequence represented by SEQ ID NO:3,
[3] a protein comprising the amino acid sequence represented by SEQ ID NO:4,
[4] a protein which consists of an amino acid sequence having 80% or more identity to the amino acid sequence represented by SEQ ID NO:2, and has the activity of uptake of the amino acid,
[5] a protein which consists of an amino acid sequence having 80% or more identity to the amino acid sequence represented by SEQ ID NO:3, and has the activity of uptake of the amino acid, and
[6] a protein which consists of an amino acid sequence having 80% or more identity to the amino acid sequence represented by SEQ ID NO:4, and has the activity of uptake of the amino acid.

2. The process according to claim 1, wherein the coryneform bacterium, in which the activity of uptake of at least one amino acid selected from the group consisting of L-arginine, L-citrulline, and L-ornithine is reduced or lost as compared with a parent strain by introducing a deletion, a substitution, or an addition of at least one nucleotide into at least one gene which is present in the chromosomal DNA of the parent strain and encodes a protein selected from the group consisting of [1] to [6] in claim 1, and the coryneform bacterium can produce the amino acid, is a coryneform bacterium in which a deletion, a substitution, or an addition of at least one nucleotide is introduced into a DNA which is present in the chromosomal DNA of the parent strain and is selected from the group consisting of the following [7] to [9],
[7] a DNA comprising the nucleotide sequence represented by SEQ ID NO:1,
[8] a DNA which hybridizes with a DNA consisting of a nucleotide sequence complementary to the nucleotide sequence represented by SEQ ID NO:1 under stringent conditions, and encodes a protein having the activity of uptake of the amino acid, and
[9] a DNA which consists of a nucleotide sequence having 80% or more identity to the nucleotide sequence represented by SEQ ID NO: 1, and encodes a protein having the activity of uptake of the amino acid.

3. The process according to claim 1 or 2, wherein the coryneform bacterium is Corynebacterium glutamicum.

4. The process according to any one of claims 1 to 3, wherein the amino acid is L-arginine or L-citrulline.


SEQUENCE LISTING

<110>  KYOWA HAKKO BIO CO., LTD.

<120>  Method for producing amino acid

<130>  1000P12253

<140>  EP13776232.4
<141>  2013-04-12

<150>  PCT/JP2013/061060
<151>  2013-04-12

<150>  JP2012-091918
<151>  2012-04-13

<160>  12

<170>  PatentIn version 3.5

<210>  1
<211>  3225
<212>  DNA
<213>  Corynebacterium glutamicum


<220>
<221>  CDS
<222>  (301)..(1164)

<220>
<221>  CDS
<222>  (1312)..(2157)

<220>
<221>  CDS
<222>  (2173)..(2925)

<400>  1
ggaaatgaga atcactgcgg caaagagcaa aatgatgaag atcgtttgtt ggctcgacat      60

agctgtttat tttaaagtcc ctcccgagcc catgccggat aaaacaaaac ccgagcgcct     120

gccaacgcaa aatccagcct cacttaaggt tattgcacca tggcaggtag tattttgccc     180

atgattgaga gtttcacccg attgcgaatt tcgcagacac cgcctctgaa acggctactg     240

catagcactt tgcgtttccc caaaatcccc aagcgcgctg tagcggcgac cgtcggcatc     300

gtg gca acc tca ttc acc ttg gct tct tgt gtc acc aat gag gag cag       348
Met Ala Thr Ser Phe Thr Leu Ala Ser Cys Val Thr Asn Glu Glu Gln
1               5                   10                  15

ggc aac cca gat ggc tgg gag cag atc gtt cca gat cct gta ccg gag       396
Gly Asn Pro Asp Gly Trp Glu Gln Ile Val Pro Asp Pro Val Pro Glu
            20                  25                  30

att cag gcg atg gtt ccc gaa gct ctg gct cag cgc ggt gtg ctc acc       444
Ile Gln Ala Met Val Pro Glu Ala Leu Ala Gln Arg Gly Val Leu Thr
        35                  40                  45

gcc ggt gcc aac cca cct ttc cca ccg ttt gag ttt aaa gat tcc gat       492

Ala Gly Ala Asn Pro Pro Phe Pro Pro Phe Glu Phe Lys Asp Ser Asp
    50                  55                  60

ggt cag att atc ggt gtg gaa atg gac ctc gtg cgt gca atg gcg ggg      540
Gly Gln Ile Ile Gly Val Glu Met Asp Leu Val Arg Ala Met Ala Gly
65                  70                  75                  80

gtg atg ggc ttg gag ttc agc cct cag gag cag gat ttc tcc ctc atc      588
Val Met Gly Leu Glu Phe Ser Pro Gln Glu Gln Asp Phe Ser Leu Ile
                85                  90                  95

ctt cca tcg gtt caa gct ggc acc ctt gat atc gga gcc tct ggc ttc      636
Leu Pro Ser Val Gln Ala Gly Thr Leu Asp Ile Gly Ala Ser Gly Phe
            100                 105                 110

act gac aac gag gaa cgc cgc gag aac ttt gat ttc atc gat ttc ctc      684
Thr Asp Asn Glu Glu Arg Arg Glu Asn Phe Asp Phe Ile Asp Phe Leu
        115                 120                 125

ttc gca ggt gtg cag tgg gcg cag gca act gat cgt gaa acc cca atc      732
Phe Ala Gly Val Gln Trp Ala Gln Ala Thr Asp Arg Glu Thr Pro Ile
    130                 135                 140

gat ccg gaa aac gcc tgt ggt ctc acc gtt gct gta cag cgc aca acc      780
Asp Pro Glu Asn Ala Cys Gly Leu Thr Val Ala Val Gln Arg Thr Thr
145                 150                 155                 160

gtg gca gag acc gac gat gtc cgt cct cgc tca gct caa tgt gaa gcc      828
Val Ala Glu Thr Asp Asp Val Arg Pro Arg Ser Ala Gln Cys Glu Ala
                165                 170                 175

gaa ggc aaa gag ccg atc acc att ttg tct tat gaa acc gca gat act      876
Glu Gly Lys Glu Pro Ile Thr Ile Leu Ser Tyr Glu Thr Ala Asp Thr
            180                 185                 190

gca gct acc gca ttg atc ctg gga cgc gca gac gca ctt gct gcg gac      924
Ala Ala Thr Ala Leu Ile Leu Gly Arg Ala Asp Ala Leu Ala Ala Asp
        195                 200                 205

tcc cct gtt tca gct tgg gct gca gag cgc tcc gaa ggc cgc atc gaa      972
Ser Pro Val Ser Ala Trp Ala Ala Glu Arg Ser Glu Gly Arg Ile Glu
    210                 215                 220

gtt gtg ggc gat atg tac ttg gct gcg cca ttt ggt ttc gca ttc ccg     1020
Val Val Gly Asp Met Tyr Leu Ala Ala Pro Phe Gly Phe Ala Phe Pro
225                 230                 235                 240

ttg gaa tct gac ctc acc cca gca gca gct gcg gcg ttc caa cac ttg     1068
Leu Glu Ser Asp Leu Thr Pro Ala Ala Ala Ala Ala Phe Gln His Leu
                245                 250                 255

att gac acc ggc gat tac cag cgc atc atg gcg caa tgg ggc att gaa     1116
Ile Asp Thr Gly Asp Tyr Gln Arg Ile Met Ala Gln Trp Gly Ile Glu
            260                 265                 270

gaa ggc ctt ctt gat gag gcc ctg atc aac gaa cag cca ctc aac tag     1164
Glu Gly Leu Leu Asp Glu Ala Leu Ile Asn Glu Gln Pro Leu Asn
        275                 280                 285

agccttccag caactaaaaa caattaaagg attacttcac agatgtcaga tcttaatcaa     1224

gggcctggag cttcaacggc ccaaccgaaa ccgattgaag ccaagccact acgccaccct     1284

ggccggtggg tcgcagccgc tatcatc gtg gca ctg ctc gca tgg ttt atc atc 1338
Met Ala Leu Leu Ala Trp Phe Ile Ile
        290                 295

agc gcg ctc aac aat gag gcc tac ggt tgg gat acc tac cgc tcg tat 1386
Ser Ala Leu Asn Asn Glu Ala Tyr Gly Trp Asp Thr Tyr Arg Ser Tyr
            300                 305                 310

ctt ttt gac acc cgc att gcc acc gcg gca ctt cac acc att gcg ctg 1434
Leu Phe Asp Thr Arg Ile Ala Thr Ala Ala Leu His Thr Ile Ala Leu
        315                 320                 325

acc ttg ctg tcc atg atc ttg ggt gtg gtt ctc ggc gca atc ttg gcc 1482
Thr Leu Leu Ser Met Ile Leu Gly Val Val Leu Gly Ala Ile Leu Ala
    330                 335                 340

gtc atg cgt atg tcc ggc aac cct gtc atg cag ggc gta gcg tgg ctg 1530
Val Met Arg Met Ser Gly Asn Pro Val Met Gln Gly Val Ala Trp Leu
345                 350                 355                 360

tac ctg tgg att ttc cgc ggc acc cca att tat gtg cag ttg gtg ttc 1578
Tyr Leu Trp Ile Phe Arg Gly Thr Pro Ile Tyr Val Gln Leu Val Phe
                365                 370                 375

tgg ggc ctg ctg ggt tcc ttg tac cag tcg atc aac ctc ggt ttc gca 1626
Trp Gly Leu Leu Gly Ser Leu Tyr Gln Ser Ile Asn Leu Gly Phe Ala
            380                 385                 390

gag atc gat ctg caa agc ttg ctg tct aat atg ttc ctg ctc gcg gtg 1674
Glu Ile Asp Leu Gln Ser Leu Leu Ser Asn Met Phe Leu Leu Ala Val
        395                 400                 405

atc ggt ctg ggt ctc aac gag gct gcg tac atg gcg gaa atc gtg cgc 1722
Ile Gly Leu Gly Leu Asn Glu Ala Ala Tyr Met Ala Glu Ile Val Arg
    410                 415                 420

tcg ggc atc caa gcg gtg cct gag ggc cag atg gag gcg tcg aaa gct 1770
Ser Gly Ile Gln Ala Val Pro Glu Gly Gln Met Glu Ala Ser Lys Ala
425                 430                 435                 440

ttg ggt atg aac tgg tca atg acc atg cgt cgc acc atc ttg ccg cag 1818
Leu Gly Met Asn Trp Ser Met Thr Met Arg Arg Thr Ile Leu Pro Gln
                445                 450                 455

gcc atg cgc atc atc att ccg cca acc ggc aat gaa ctg atc tcc atg 1866
Ala Met Arg Ile Ile Ile Pro Pro Thr Gly Asn Glu Leu Ile Ser Met
            460                 465                 470

ctc aag acc acc tct ctg gtt gtt gcg att cct tat tct ctc gag ctg 1914
Leu Lys Thr Thr Ser Leu Val Val Ala Ile Pro Tyr Ser Leu Glu Leu
        475                 480                 485

tac ggc cgc agc atg gat att gcg tac tcc ctc ttc gag cca gtt cca 1962
Tyr Gly Arg Ser Met Asp Ile Ala Tyr Ser Leu Phe Glu Pro Val Pro
    490                 495                 500

atg ctt ctg gtt gct gcg agc tgg tac ttg gtc atc acc tct att ctt 2010
Met Leu Leu Val Ala Ala Ser Trp Tyr Leu Val Ile Thr Ser Ile Leu
505                 510                 515                 520

atg gtt ggt cag tac tac ctg gag aag cac ttc gaa aag ggc agc acc 2058
Met Val Gly Gln Tyr Tyr Leu Glu Lys His Phe Glu Lys Gly Ser Thr
                525                 530                 535

cgc acc ctg acc gca cgt cag ctc gct gcg ttg gct gat gct gaa ggc 2106
Arg Thr Leu Thr Ala Arg Gln Leu Ala Ala Leu Ala Asp Ala Glu Gly
            540                 545                 550

gca atc ccg ggc aat gtc acc gtt gtt cct gaa act tcc aag gag aac 2154
Ala Ile Pro Gly Asn Val Thr Val Val Pro Glu Thr Ser Lys Glu Asn
        555                 560                 565

tag caaatgtcgc aactg atg att gat gct cag cag gtc tgc aaa aac tac 2205
Met Ile Asp Ala Gln Gln Val Cys Lys Asn Tyr
    570                 575

gga cgc ttg gaa gtg ctc aag ggc att gac ttg cag gtt cct aaa ggt 2253
Gly Arg Leu Glu Val Leu Lys Gly Ile Asp Leu Gln Val Pro Lys Gly
580                 585                 590                 595

acc gtc acc tgc ttg atc ggt cct tct ggt tcc ggt aaa tcc acc atg 2301
Thr Val Thr Cys Leu Ile Gly Pro Ser Gly Ser Gly Lys Ser Thr Met
                600                 605                 610

ctg cgc tgc gtt aac cac ctg gaa aag gtc aac gcg ggt cgc ttg tat 2349
Leu Arg Cys Val Asn His Leu Glu Lys Val Asn Ala Gly Arg Leu Tyr
            615                 620                 625

gtc gat ggc gat ctc att ggc tac cgc gag cgc gat ggc gtg ctg tac 2397
Val Asp Gly Asp Leu Ile Gly Tyr Arg Glu Arg Asp Gly Val Leu Tyr
        630                 635                 640

gaa atc tct gag aag gac gcc gcc aag cag cgc tcc gat atc ggc atg 2445
Glu Ile Ser Glu Lys Asp Ala Ala Lys Gln Arg Ser Asp Ile Gly Met
    645                 650                 655

gtg ttc cag aac ttc aac ctc ttc ccc cac cgc acg gtg atc gag aac 2493
Val Phe Gln Asn Phe Asn Leu Phe Pro His Arg Thr Val Ile Glu Asn
660                 665                 670                 675

atc atc gaa gct ccc atc cac gtg aag aag cag ccc gaa agc aag gcc 2541
Ile Ile Glu Ala Pro Ile His Val Lys Lys Gln Pro Glu Ser Lys Ala
                680                 685                 690

cgc gca cgt gcc atg gag ctg ctt gag cag gtc ggc ctc gcc cac aag 2589
Arg Ala Arg Ala Met Glu Leu Leu Glu Gln Val Gly Leu Ala His Lys
            695                 700                 705

gcg gac gcc tac ccc gtc caa ctg tcg ggt ggt cag cag cag cgc gtt 2637
Ala Asp Ala Tyr Pro Val Gln Leu Ser Gly Gly Gln Gln Gln Arg Val
        710                 715                 720

gca att gcc cgc gcc gtc gcc atg gag cca aag ctc atg ctt ttc gac 2685
Ala Ile Ala Arg Ala Val Ala Met Glu Pro Lys Leu Met Leu Phe Asp
    725                 730                 735

gaa ccc acc agc gct ttg gac cct gaa ctc gtc ggt gaa gtc ctg cga 2733
Glu Pro Thr Ser Ala Leu Asp Pro Glu Leu Val Gly Glu Val Leu Arg
740                 745                 750                 755

gtg atg aaa cag ctc gcc gac gac ggc atg acc atg ctt gtt gtc acc 2781
Val Met Lys Gln Leu Ala Asp Asp Gly Met Thr Met Leu Val Val Thr
                760                 765                 770

cac gaa atg ggc ttc gcc cac gaa gtc gcc gac cag gtc gtg ttc atg 2829
His Glu Met Gly Phe Ala His Glu Val Ala Asp Gln Val Val Phe Met

775                 780                 785

gcc gat gga gtt gtc gtt gaa gcc gga acc ccc gaa caa gtt ctg gac      2877
Ala Asp Gly Val Val Val Glu Ala Gly Thr Pro Glu Gln Val Leu Asp
        790                 795                 800

aat cca aag gaa cag cgc acc aaa gac ttc ctg tct tct ctg ctc taa      2925
Asn Pro Lys Glu Gln Arg Thr Lys Asp Phe Leu Ser Ser Leu Leu
    805                 810                 815

ccttttcggg tcttaaaaaa cctgcgagtc attatcggct cgcaggtttt tgcgttcaag      2985

ataacccaag aactcggaaa tgaaggcatc ccactgatac tggcgcccct ggtgagactc      3045

gaactcacac tggacgggtt tttgaatcca ttaaccgtcc catcgagacc aagggagctt      3105

actgccgacg aggttttggt ctcgatctgc agtatttcag cctgtaaacg cgattttcag      3165

accaagggac gctcagtcac ttagggattt ggcctaattc tgacggttgc ggaaagctgc      3225

<210>  2
<211>  287
<212>  PRT
<213>  Corynebacterium glutamicum

<400>  2

Met Ala Thr Ser Phe Thr Leu Ala Ser Cys Val Thr Asn Glu Glu Gln
1               5                   10                  15

Gly Asn Pro Asp Gly Trp Glu Gln Ile Val Pro Asp Pro Val Pro Glu
            20                  25                  30

Ile Gln Ala Met Val Pro Glu Ala Leu Ala Gln Arg Gly Val Leu Thr
        35                  40                  45

Ala Gly Ala Asn Pro Pro Phe Pro Pro Phe Glu Phe Lys Asp Ser Asp
    50                  55                  60

Gly Gln Ile Ile Gly Val Glu Met Asp Leu Val Arg Ala Met Ala Gly
65                  70                  75                  80

Val Met Gly Leu Glu Phe Ser Pro Gln Glu Gln Asp Phe Ser Leu Ile
                85                  90                  95

Leu Pro Ser Val Gln Ala Gly Thr Leu Asp Ile Gly Ala Ser Gly Phe
            100                 105                 110

Thr Asp Asn Glu Glu Arg Arg Glu Asn Phe Asp Phe Ile Asp Phe Leu
        115                 120                 125

Phe Ala Gly Val Gln Trp Ala Gln Ala Thr Asp Arg Glu Thr Pro Ile
    130                 135                 140

Asp Pro Glu Asn Ala Cys Gly Leu Thr Val Ala Val Gln Arg Thr Thr
145                 150                 155                 160

Val Ala Glu Thr Asp Asp Val Arg Pro Arg Ser Ala Gln Cys Glu Ala
                165                 170                 175

Glu Gly Lys Glu Pro Ile Thr Ile Leu Ser Tyr Glu Thr Ala Asp Thr
            180                 185                 190

Ala Ala Thr Ala Leu Ile Leu Gly Arg Ala Asp Ala Leu Ala Ala Asp
        195                 200                 205

Ser Pro Val Ser Ala Trp Ala Ala Glu Arg Ser Glu Gly Arg Ile Glu
    210                 215                 220

Val Val Gly Asp Met Tyr Leu Ala Ala Pro Phe Gly Phe Ala Phe Pro
225                 230                 235                 240

Leu Glu Ser Asp Leu Thr Pro Ala Ala Ala Ala Ala Phe Gln His Leu
                245                 250                 255

Ile Asp Thr Gly Asp Tyr Gln Arg Ile Met Ala Gln Trp Gly Ile Glu
            260                 265                 270

Glu Gly Leu Leu Asp Glu Ala Leu Ile Asn Glu Gln Pro Leu Asn
        275                 280                 285

<210>  3
<211>  281
<212>  PRT
<213>  Corynebacterium glutamicum

<400>  3

Met Ala Leu Leu Ala Trp Phe Ile Ile Ser Ala Leu Asn Asn Glu Ala
1               5                   10                  15

Tyr Gly Trp Asp Thr Tyr Arg Ser Tyr Leu Phe Asp Thr Arg Ile Ala
            20                  25                  30

Thr Ala Ala Leu His Thr Ile Ala Leu Thr Leu Leu Ser Met Ile Leu
        35                  40                  45

Gly Val Val Leu Gly Ala Ile Leu Ala Val Met Arg Met Ser Gly Asn
    50                  55                  60

Pro Val Met Gln Gly Val Ala Trp Leu Tyr Leu Trp Ile Phe Arg Gly
65                  70                  75                  80

Thr Pro Ile Tyr Val Gln Leu Val Phe Trp Gly Leu Leu Gly Ser Leu
                85                  90                  95

Tyr Gln Ser Ile Asn Leu Gly Phe Ala Glu Ile Asp Leu Gln Ser Leu
            100                 105                 110

Leu Ser Asn Met Phe Leu Leu Ala Val Ile Gly Leu Gly Leu Asn Glu
        115                 120                 125

Ala Ala Tyr Met Ala Glu Ile Val Arg Ser Gly Ile Gln Ala Val Pro
    130                 135                 140

Glu Gly Gln Met Glu Ala Ser Lys Ala Leu Gly Met Asn Trp Ser Met
145                 150                 155                 160

Thr Met Arg Arg Thr Ile Leu Pro Gln Ala Met Arg Ile Ile Ile Pro
                165                 170                 175

Pro Thr Gly Asn Glu Leu Ile Ser Met Leu Lys Thr Thr Ser Leu Val
            180                 185                 190

Val Ala Ile Pro Tyr Ser Leu Glu Leu Tyr Gly Arg Ser Met Asp Ile
        195                 200                 205

Ala Tyr Ser Leu Phe Glu Pro Val Pro Met Leu Leu Val Ala Ala Ser
    210                 215                 220

Trp Tyr Leu Val Ile Thr Ser Ile Leu Met Val Gly Gln Tyr Tyr Leu
225                 230                 235                 240

Glu Lys His Phe Glu Lys Gly Ser Thr Arg Thr Leu Thr Ala Arg Gln
                245                 250                 255

Leu Ala Ala Leu Ala Asp Ala Glu Gly Ala Ile Pro Gly Asn Val Thr
            260                 265                 270

Val Val Pro Glu Thr Ser Lys Glu Asn
        275                 280

<210>  4
<211>  250
<212>  PRT
<213>  Corynebacterium glutamicum

<400>  4

Met Ile Asp Ala Gln Gln Val Cys Lys Asn Tyr Gly Arg Leu Glu Val
1               5                   10                  15

Leu Lys Gly Ile Asp Leu Gln Val Pro Lys Gly Thr Val Thr Cys Leu
            20                  25                  30

Ile Gly Pro Ser Gly Ser Gly Lys Ser Thr Met Leu Arg Cys Val Asn
        35                  40                  45

His Leu Glu Lys Val Asn Ala Gly Arg Leu Tyr Val Asp Gly Asp Leu
    50                  55                  60

Ile Gly Tyr Arg Glu Arg Asp Gly Val Leu Tyr Glu Ile Ser Glu Lys
65                  70                  75                  80

Asp Ala Ala Lys Gln Arg Ser Asp Ile Gly Met Val Phe Gln Asn Phe
                85                  90                  95

Asn Leu Phe Pro His Arg Thr Val Ile Glu Asn Ile Ile Glu Ala Pro
            100                 105                 110

Ile His Val Lys Lys Gln Pro Glu Ser Lys Ala Arg Ala Arg Ala Met
        115                 120                 125

Glu Leu Leu Glu Gln Val Gly Leu Ala His Lys Ala Asp Ala Tyr Pro
    130                 135                 140

Val Gln Leu Ser Gly Gly Gln Gln Gln Arg Val Ala Ile Ala Arg Ala
145                 150                 155                 160

Val Ala Met Glu Pro Lys Leu Met Leu Phe Asp Glu Pro Thr Ser Ala
                165                 170                 175

Leu Asp Pro Glu Leu Val Gly Glu Val Leu Arg Val Met Lys Gln Leu
            180                 185                 190

Ala Asp Asp Gly Met Thr Met Leu Val Val Thr His Glu Met Gly Phe
        195                 200                 205

Ala His Glu Val Ala Asp Gln Val Val Phe Met Ala Asp Gly Val Val
    210                 215                 220

Val Glu Ala Gly Thr Pro Glu Gln Val Leu Asp Asn Pro Lys Glu Gln
225                 230                 235                 240

Arg Thr Lys Asp Phe Leu Ser Ser Leu Leu
                245                 250

<210>  5
<211>  21

<212>  DNA
<213>  Artificial Sequence

<220>
<223>  Synthetic DNA

<400>  5
aaggcagtgc tgtctgttgt c                                                 21


<210>  6
<211>  19
<212>  DNA
<213>  Artificial Sequence

<220>
<223>  Synthetic DNA

<400>  6
atgcggcgca gcttctccg                                                    19


<210>  7
<211>  33
<212>  DNA
<213>  Artificial Sequence

<220>
<223>  Synthetic DNA

<400>  7
atatatggat ccgccaacgc aaaatccagc ctc                                    33


<210>  8
<211>  42
<212>  DNA
<213>  Artificial Sequence

<220>
<223>  Synthetic DNA

<400>  8
cccatccact aaacttaaac aaaccgatgg aaggatgagg ga                          42


<210>  9
<211>  44
<212>  DNA
<213>  Artificial Sequence

<220>
<223>  Synthetic DNA

<400>  9
tgtttaagtt tagtggatgg gccgatcacc attttgtctt atga                        44


<210>  10
<211>  34
<212>  DNA
<213>  Artificial Sequence

<220>
<223>  Synthetic DNA

<400>  10
atatatggat cccgctgatg ataaaccatg cgag                                34


<210>  11
<211>  32
<212>  DNA
<213>  Artificial Sequence

<220>
<223>  Synthetic DNA

<400>  11
atatatgtcg actgtcgtgg agcaagctcc ag                                  32


<210>  12
<211>  32
<212>  DNA
<213>  Artificial Sequence

<220>
<223>  Synthetic DNA

<400>  12
atatatggta ccgtcatcag tgacggttgc gc                                  32